# EUROPÄISCHE PATENTSCHRIFT

(11) **EP 0 457 017 B1**
(45) Veröffentlichungstag und Bekanntmachung des Hinweises auf die Patenterteilung: **20.09.1995**
(21) Anmeldenummer: 91105485.6
(22) Anmeldetag: 06.04.1991
(51) Int. Cl.: A61B 17/58, A61B 17/28

(54) **Klammerfixationssystem**
Fixations system for clamps
Système de fixation pour une pince

(30) Priorität: 15.05.1990 CH 1640/90
(43) Veröffentlichungstag der Anmeldung: 21.11.1991
(73) Patentinhaber: Synthes AG, Chur, CH-7002 Chur (CH)
(72) Erfinder: Frigg, Robert, CH-7270 Davos-Platz (CH)
(74) Vertreter: Lusuardi, Werther Giovanni, Dr.

(56) Entgegenhaltungen:
- CH-A- 492 443
- FR-A- 752 676
- FR-A- 2 557 933
- US-A- 1 635 137
- US-A- 2 427 128
- US-A- 2 631 585
- US-A- 4 475 544

## Beschreibung

Die Erfindung bezieht sich auf eine Knochenfasszange gemäss dem Oberbegriff des Anspruchs 1 sowie auf eine osteosynthetische Fixationsvorrichtung gemäss dem Oberbegriff des Anspruchs 8.

Zur Behandlung von Knochenbrüchen, insbesondere zur Fixierung von Knochen oder Knochenfragmenten, stehen dem Chirurgen eine Vielzahl von Osteosynthesemitteln zur Verfügung.
In den letzten Jahren konnte man eindeutig die Tendenz von der konservativen zur operativen Frakturversorgung beobachten. Dabei stand zu Beginn der operativen Frakturbehandlung die optimale Rekonstruktion der Fraktur im Vordergrund. Der Blutversorgung und den Weichteilen rund um die Fraktur wurde keine grosse Beachtung geschenkt. Das Ziel der absoluten Stabilität wurde ohne Rücksicht auf die Biologie angestrebt. Mit der Verbreitung der geschlossenen Marknagelung wurde erkannt, dass eine absolute Stabilität der Fraktur nicht unbedingt erforderlich ist. Trotz zum Teil grosser Instabilität der Fraktur, vermochte die nur minimal gestörte Biologie die Fraktur zu heilen. Diese neu erlangte Kenntnis, wurde auch bei anderen Osteosynthesearten berücksichtigt. Bei der Plattenosteosynthese z.B. werden weniger Schrauben, kleinere und kürzere Platten verwendet. Das gleiche gilt auch beim Fixateur externe. An Stelle von riesigen, mehrdimensionalen Konstruktionen, wenn möglich noch mit interfragmentärer Zugschraube, setzte sich der einfache unilaterale Fixateur durch.

Trotz der "biologischen" Anwendung des Fixateur externe gelingt eine Ausheilung der Fraktur nicht immer. Das heisst, oft muss die Fraktur mit einem zweiten Eingriff und einer anderen Osteosynthesemethode behandelt werden. Der Hauptgrund für die Komplikationen sind die Nägel oder Schrauben, die von aussen durch die Haut und die Weichteile in den Knochen eingebracht werden. Entlang dieser Nägel oder Schrauben gelangen Keime bis in den Knochen, was zu einer sogenannten "Pintrak-Infektion" führt. Ist eine derartige Infektion vorhanden, müssen die Nägel oder Schrauben entfernt werden. Während einer Heilungszeit der Infektion von ca. 10 Tagen kann die Fraktur nicht operativ fixiert werden. Das hat zur Folge, dass der Patient während dieser Zeit immobilisiert werden muss.
Neben diesem Nachteil des herkömmlichen Fixateur externe, zeichnet sich ein weiterer Nachteil immer deutlicher ab. Messungen mit Laserdopplern, mit denen die Durchblutung des Knochens klinisch gemessen werden kann, haben gezeigt, dass das Einbringen von Nägeln oder Schrauben die Durchblutung des Knochens erheblich stört. Für den Kliniker bedeutet das, dass er schon beim Einbringen von Fixateur externe, Nägeln oder Schrauben den weiteren Behandlungsablauf vorgibt (Gefahr von Pintrak-Infektion, 10-tägige Immobilisation des Patienten bei Verfahrenswechsel). Negativ wirkt sich diese Tatsache dann aus, wenn der Fixateur externe aus zeitlichen Gründen, oder zur momentanen Behandlung von Weichteilschäden benutzt wird. Stellt man sich die Situation einer Katastrophe vor (Flugzeugabsturz, Zugsunglück usw.), so müssen die Verletzten schnellstmöglich behandelt werden. Für solche Fälle bietet sich der Fixateur externe vorzüglich an. Die vorher beschriebenen Probleme werden jedoch auch hier nicht ausbleiben.

Aus der US-A-1635137 MULLENS ist eine Knochenklammer bekannt, bei welcher zwei miteinander gekoppelte gattungsgemässe Knochenfasszangen mittels einer gemeinsamen zentralen Feststellvorrichtung in lösbarer Weise blockiert werden können. Wegen der beiden miteinander gekoppelten Zangen ist diese Vorrichtung voluminös und schwierig zu handhaben. Ein weiterer Nachteil liegt darin begründet, dass die nur gleichzeitig auf beide Zangen wirkende Feststellvorrichtung in einem ziemlich grossen Abstand von der gemeinsamen Gelenkachse der beiden Knochenfasszangen angebracht ist, was ein weiteres Anwachsen des Volumens der Vorrichtung zur Folge hat.

Hier will die Erfindung Abhilfe schaffen. Der Erfindung liegt die Aufgabe zugrunde, eine einfache, manuell bedienbare Knochenfasszange kompakter Bauart zu schaffen, welche eine nicht-invasive, temporäre Fixation von Knochenfrakturen und das Verbinden mehrerer solcher Knochenfasszangen miteinander zu einer stabilen osteosynthetischen Fixationsvorrichtung gestattet.

Die Erfindung löst die gestellte Aufgabe mit einer Knochenfasszange, welche die Merkmale des Anspruchs 1 aufweist, sowie einer osteosynthetischen Fixationsvorrichtung, welche die Merkmale des Anspruchs 8 aufweist.

An Stelle einer Schraube oder eines Nagels, wird der Knochen zwischen den vorzugsweise spitz ausgestalteten Lippen der erfindungsgemässen Knochenfasszange gehalten. Das hat den Vorteil, dass der Knochen nicht durchbohrt werden muss und dadurch keine Hitzenekrose durch den Bohrer hervorgerufen wird und dass die Durchblutung des Knochens in minimalster Weise gestört wird. Mehrere solcher zangenartiger Knochenfasszangen können gleich wie Schrauben oder Nägel extern miteinander verbunden werden. Die Hauptindikation der erfindungsgemässen Knochenfasszange ist die temporäre Fixation von offenen Unterschenkelfrakturen. Das heisst, sie können lediglich zur intraoperativen Reposition der Fraktur verwendet werden, oder nach erfolgter Reposition solange am Knochen belassen werden, bis die Weichteilheilung abgeschlossen ist. In beiden Fällen kann bei liegenden Knochenfasszangen ein Verfahrenswechsel durchgeführt werden (z.B. Marknagelung). Die Belassung der Knochenfasszangen bis zur Ausheilung der Fraktur ist problemlos möglich, aber wegen der einfachen Möglichkeit eines Verfahrenswechsels selten sinnvoll.
Sollte es während der Behandlungszeit zu einer "Pintrak-Infektion" kommen, ist diese nur oberflächlich und kann einfach behandelt werden, da der Knochen unterhalb der Spitzen der Knochenfasszange vital ist.

Die Anwendung der erfindungsgemässen Knochenfasszange beschränkt sich nicht nur auf die klassische "Fixateur externe Methode", sondern kommt überall dort zum Einsatz, wo eine Fraktur reponiert oder die Knochenlänge und Rotation gehalten werden muss.
Eine nutzbringende Anwendung der erfindungsgemässen Knochenfasszange besteht überdies in der temporären Verriegelung eines Marknagels, da das Anbringen der erfindungsgemässen Knochenfasszange im Vergleich zu Schrauben viel einfacher, schneller und ohne Verwendung von Röntgenstrahlen durchgeführt werden kann. Das gleiche gilt bei der indirekten Reposition mit dem Distraktor. Anstelle von Schanzschen Schrauben können die erfindungsgemässen Knochenfasszangen verwendet werden, um die für die Frakturversorgung unnötigen Bohrungen im Knochen zu vermeiden.

Enorme Vorteile bietet die erfindungsgemässe Knochenfasszange bei der Behandlung von Kriegsverletzten oder in Katastrophenfällen, wo die Frakturversorgung ohne grosszügige Hilfsmittel, schnell und zum Teil von minimal geschulten Personen durchgeführt werden muss. Bei diesen Gegebenheiten bietet die erfindungsgemässe Knochenfasszange ein viel geringeres Infektionsrisiko, und es werden keine zusätzlichen Instrumente, Maschinen usw. benötigt. Ungenügend gut versorgte Frakturen können, in einem späteren Zeitpunkt, problemlos korrigiert oder neu fixiert werden.

Die örtliche Anwendung der erfindungsgemässen Knochenfasszangen hängt von den Weichteilstrukturen ab (Blutgefässe, Nerven usw.), die den Knochen umschliessen. Sind gefährdete Strukturen vorhanden, muss der Zugang zum Knochen, wie bei der Anwendung des Fixateurs externe, vorsichtig präpariert werden.

Die Ausbildung der Erfindung in Form einer Zange hat vielerlei Vorteile.

Die erfindungsgemässe Knochenfasszange kann wie andere zangenartige Instrumente vom Chirurgen gehandhabt werden. Der Chirurg ist somit bereits im Umgang mit solchen Geräten geübt, und weiss, wie die Klemmkraft der Knochenfasszange dosiert werden kann. Auch ist das positionsrichtige Ansetzen einer Zange viel einfacher als z.B. das richtige Ansetzen einer Spannzwinge.
Die Zangenlippen sind vorzugsweise so konstruiert, dass sie eine federnde Eigenschaft haben. Das hat den Vorteil, dass bei eventuellem Einsinken oder leichtem Abrutschen der Zangenspitzen am Knochen, kein merklicher Kraftverlust der Zange auf den Knochen auftritt.

Der Zangenverschluss befindet sich im Zangenschloss und kann durch einfaches Festdrehen einer Mutter desaktiviert werden. Das hat den Vorteil, dass die Handgriffe nach erfolgreicher Fixation der Knochenfasszange am Knochen entfernt werden können, um so die externe Konstruktion so klein wie möglich zu gestalten.

Der um die Achse des Gelenkachselementes rotierbar angeordnete Verbindungsstift ermöglicht die Verbindung von mehreren, einzeln gesetzten Knochenfassvorrichtungen zu einem geraden Verbindungsrohr.

Ein Ausführungsbeispiel der Erfindung, welches zugleich das Funktionsprinzip erläutert, ist in der Zeichnung dargestellt und wird im folgenden näher beschrieben.
Fig. 1 stellt eine Aufsicht auf die erfindungsgemässe Knochenfasszange dar;
Fig. 2 stellt eine Ansicht von unten der Knochenfasszange mit einem Verbindungsstift dar;
Fig. 3 stellt eine perspektivische Darstellung der Knochenfasszange nach Fig. 1 dar; und
Fig. 4 stellt eine Explosionszeichnung der Knochenfasszange nach Fig. 2 dar.

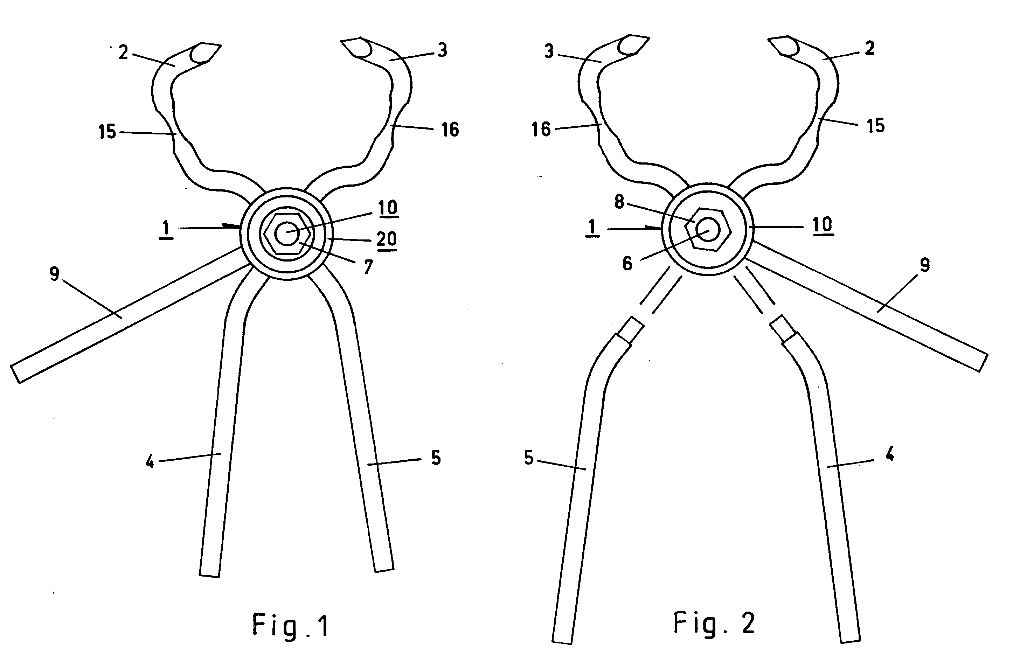

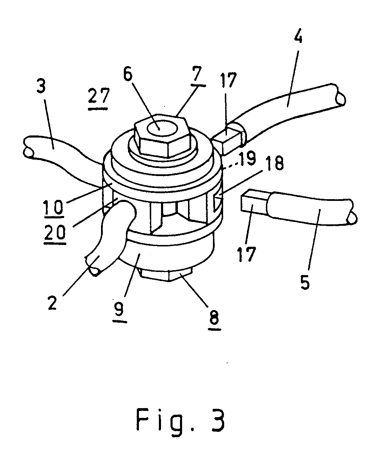

Die in den Fig. 1 und 2 je von oben und von unten dargestellte Knochenfasszange besteht im wesentlichen aus einem Drehgelenk 1, zwei Lippen 2,3 und zwei Schenkeln 4,5. Das Drehgelenk 1 kann durch manuelle Betätigung der Schenkel 4,5 betätigt werden, wodurch sich die Lippen 2,3 gegeneinander bewegen.
Die beiden kieferförmig gebogenen Lippen 2,3 sind durch eine Querschnittsverengung 15, resp. 16 federnd ausgebildet, so dass der Chirurg beim Fassen eines Knochenfragmentes eine manuell dosierte federnde Kraft ausüben kann. Die freien Enden der beiden Lippen 2,3 sind spitz ausgebildet, um eine sichere Erfassung der Knochenfragmente zu gewährleisten.

Nach erfolgter Erfassung des, resp. der erwünschten Knochenfragmente mittels der Feststellvorrichtung 7 (hier als Feststellmutter realisiert, welche einfach zugeschraubt werden kann), das Drehgelenk 1, bzw. seine beiden Elemente, nämlich das Gelenkachselement 10 und das Gelenkbohrungselement 20, gegeneinander blockiert werden, wobei die vom Chirurgen aufgebrachte Federspannung bestehen bleibt und eine Lockerung der Zangenspannung verhindert. Durch einfaches Lösen der Feststellvorrichtung 7 (Losschrauben der Feststellmutter) lässt sich das Drehgelenk 1 wieder deblockieren. Nachdem die Knochenfasszange am gewünschten Ort mit der gewünschten Federspannung fixiert worden ist, können die beiden als Handgriffe ausgebildeten Schenkel 4,5 vom Gelenk 1 entfernt werden. Zu diesem Zweck sind die Schenkel 4,5 - wie in Fig. 3 dargestellt - an ihrem gelenkseitigen Ende vorzugsweise mit einem Vierkant 17 versehen, der in korrespondierende, radial angeordnete Öffnungen 18 (im Gelenkbohrungselement 20) und 19 (im Gelenkachselement 10) eingesteckt und daraus wieder entfernt werden kann. Diese Massnahme gestattet es, den osteosynthetisch wirksamen Teil der Knochenfasszange (Fixationsklammer) möglichst klein zu halten.

Wie in Fig. 2 dargestellt, weist die Knochenfasszange einen um die Achse 6 des Gelenkachselementes 10 rotierbar angeordneten, mittels einer Feststellmutter 8 in jeder beliebigen Position blockierbaren Verbindungsstift 9 auf. Dieser Verbindungsstift 9 kann entweder schon präoperativ angebracht werden oder auch erst intraoperativ, falls die räumlichen Verhältnisse dies nahelegen. Mittels dieses Verbindungsstifts 9 ist es möglich, eine ganze Serie von Knochenfasszangen untereinander zu verbinden, um damit einen grösseren Verbund herzustellen. Die Verbindung der Stifte 9 untereinander kann auf konventionelle Weise erfolgen mit den üblichen Hilfsmitteln (Stäbe, Klemmen u.ä.), wie sie bei Fixateurs externes angewendet werden.

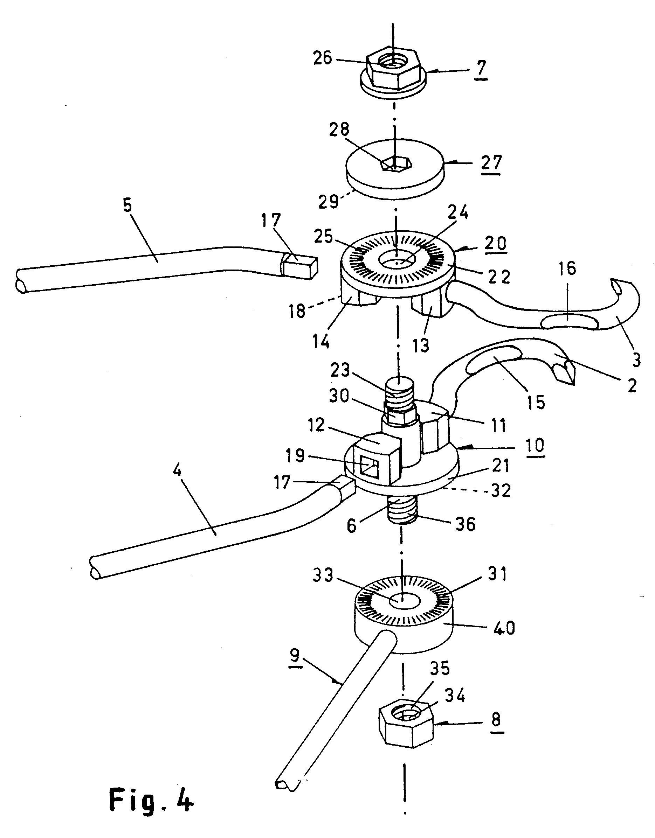

Wie in Fig. 4 dargestellt, besteht das Drehgelenk 1 bei einer bevorzugten Ausführungsform aus einem Achsengelenk mit einem Gelenkachselement 10 und einem mit dem Gelenkachselement 10 korrespondierenden Gelenkbohrungselement 20. Die beiden Elemente 10,20 weisen tellerförmige Grundkörper 21,22 auf, welche je eine der beiden Lippen 2,3 tragen. Der Grundkörper 21 des Gelenkachselementes 10 trägt eine axial angeordnete zylindrischen Gelenkachse 6, welche an ihren beiden Enden mit je einem Gewinde 23,24 versehen ist. Der Grundkörper 22 des Gelenkbohrungselementes 20 weist eine zentrale Bohrung 24 auf, welche dazu bestimmt ist, die zylindrische Gelenkachse 6 aufzunehmen.

Die beiden tellerförmigen Grundkörper 21,22 sind mit je zwei konzentrisch gegeneinander versetzt angeordneten Bewegungsbegrenzern (11,12;13,14) versehen, welche die gegenseitige Rotation der beiden Elemente 10,20 in positiver und negativer Drehrichtung begrenzen.

Um die Position des Gelenkachselementes 10 gegenüber dem Gelenkbohrungselement 20 in lösbarer Weise fixierbar zu gestalten (Blockierung des Gelenkes 1), ist, neben der als Feststellvorrichtung 7 dienenden, eine Bohrung 26 aufweisenden Feststellschraube, eine zwischen letzterer und dem Gelenkbohrungselement 20 zu liegen kommende Zwischenscheibe 27 vorgesehen, welche im Zentrum eine sechskantige Öffnung 28 und an ihrer Unterseite eine konzentrisch angeordnete gerillte Kreiszone 29 aufweist. Der Grundkörper 22 des Gelenkbohrungselementes 20 weist an seiner Oberseite eine mit der Kreiszone 29 der Zwischenscheibe 27 korrespondierende, gerillte Kreiszone 25 auf. Schliesslich ist derjenige Teil der Gelenkachse 6, der auf Höhe der Zwischenscheibe 27 zu liegen kommt, mit einem, einen sechskantigen Querschnitt aufweisenden Abschnitt 30 ausgebildet, um bei einer Drehung der Gelenkachse 6 die Zwischenscheibe 27 mitrotieren zu lassen. Diese Konstruktion erlaubt es, bei losgeschraubter Feststellvorrichtung 7 die beiden Elemente 10 und 20 gegeneinander zu bewegen (die beiden gerillten Kreisringe 25,29 stehen nicht miteinander im Eingriff), währenddem bei festgeschraubter Feststellvorrichtung 7 die beiden Kreiszonen 25 und 29 mit ihren Strukturierungen gegeneinander festgeklemmt sind, so dass die Zwischenscheibe 27 und damit auch der in der sechskantigen Öffnung 28 festsitzende Abschnitt 30 der Gelenkachse 6 gegenüber dem Gelenkbohrungsteil 20 blockiert ist.

Mittels einer ähnlichen Konstruktion ist es möglich, den Verbindungsstift 9 in seiner radialen Position zu fixieren. Zu diesem Zweck ist der Verbindungsstift 9 mit einem tellerförmigen Grundkörper 40 versehen, der an seiner Oberseite eine kreisringförmige, gerillte Zone 31 aufweist, welche mit einer an der Unterseite des tellerförmigen Grundkörpers 21 des Gelenkachselementes 10 befindlichen gerillten Kreiszone 32 korrespondiert.
Der tellerförmige Grundkörper 40 weist eine zentrisch angeordnete Bohrung 33 auf, welche die Gelenkachse 6 aufnehmen kann. Mittels der Sechskant-Mutter 8, welche eine zentrische Bohrung 34 mit Innengewinde 35 aufweist, kann der Verbindungsstift 9 mit der Gelenkachse 6 verbunden werden (das Innengewinde 35 der Bohrung 34 korrespondiert mit dem Aussengewinde 36 der Gelenkachse 6).
Wird die Mutter 8 nur teilweise auf das Aussengewinde 36 aufgeschraubt, so kann der Verbindungsstift 9 beliebig um die Achse 6 des Gelenkachselementes 10 rotiert werden; durch Festziehen der Mutter 8 verzahnen sich die beiden gerillten Kreiszonen 31 und 32, so dass der Verbindungsstift 9 in jeder beliebigen Position blockiert werden kann.

Dank des Verbindungsstiftes 9 besteht die Möglichkeit, zwei oder mehr Knochenfasszangen mittels in der Osteosynthese üblicher Hilfselemente (Klammern, Zwingen u.ä.) an Stäbe eines Fixateurs externe zu befestigen, um auf diese Weise ein äusserst stabiles Gebilde zu erhalten.

## Patentansprüche

1. Knochenfasszange mit
A) einem, ein Gelenkachselement (10) und ein mit dem Gelenkachselement (10) korrespondierendes Gelenkbohrungselement (20) umfassenden Gelenk (1), wobei das Gelenkachselement (10) eine axial angeordnete, zylindrische Gelenkachse (6) trägt;
B) zwei Lippen (2,3);
C) zwei Schenkeln (4,5); und
D) einer Feststellvorrichtung (7) für das Gelenk (1) mit welcher die Position des Gelenkachselementes (10) gegenüber dem Gelenkbohrungselement (20) in lösbarer Weise fixierbar ist; **dadurch gekennzeichnet**, dass
E) die Feststellvorrichtung (7) direkt an der zylindrischen Gelenkachse (6) des Gelenkachselementes (10) angreift;
F) ein Verbindungsstift (9) vorgesehen ist, mit welchem eine Verbindung zu weiteren Knochenfasszangen herstellbar ist; und
G) der Verbindungsstift (9) um die Achse (6) des Gelenkachselementes (10) rotierbar angeordnet ist und mittels einer Fixationsvorrichtung (8) in jeder beliebigen Position blockierbar ist.

2. Knochenfasszange nach Anspruch 1, dadurch gekennzeichnet, dass mindestens eine der Lippen (2;3) federnd (15;16) ausgebildet ist.

3. Knochenfasszange nach Anspruch 1 oder 2, dadurch gekennzeichnet, dass die beiden, vorzugsweise als Handgriffe ausgebildeten Schenkel (4,5) vom Gelenk (1) entfernbar sind.

4. Knochenfasszange nach einem der Ansprüche 1 bis 3, dadurch gekennzeichnet, dass das freie Ende der beiden Lippen (2,3) spitz ausgebildet ist.

5. Knochenfasszange nach einem der Ansprüche 1 bis 4, dadurch gekennzeichnet, dass die Feststellvorrichtung (7) eine Feststellmutter ist.

6. Knochenfasszange nach Anspruch 5, dadurch gekennzeichnet, dass das Gelenkachselement (10) und das Gelenkbohrungselement (20) mit je zwei konzentrisch gegeneinander versetzt angeordneten Bewegungsbegrenzern (11,12;13,14) versehen sind, welche die gegenseitige Rotation der beiden Elemente (10,20) in positiver und negativer Drehrichtung begrenzen.

7. Knochenfasszange nach einem der Ansprüche 1 bis 6, dadurch gekennzeichnet, dass die Fixationsvorrichtung (8) eine Feststellmutter (8) ist.

8. Osteosynthetische Fixationsvorrichtung mit mindestens zwei Knochenfasszangen nach einem der Ansprüche 1 - 7, dadurch gekennzeichnet, dass die einzelnen Knochenfasszangen über ihre Verbindungsstifte (9) miteinander verbunden sind.

## Claims

1. Bone gripping pincers with
A) a hinge (1) having a hinge pin component (10) and a hinge bore component (20) corresponding with the hinge pin component (10), whereby the hinge pin component (10) is bearing an axially arranged cylindrical hinge axis (6);
B) two jaws (2,3);
C) two legs (4,5); and
D) a locking device (7) for the hinge (1) which allows to releasably fix the position of the hinge pin component (10) with regard to the hinge bore component (20);
**characterized in that**
E) the locking device (7) is acting directly upon the cylindrical hinge axis (6) of the hinge pin component (10);
F) a connection pin (9) is provided allowing the connection with a further bone gripping pincers; and
G) the connection pin (9) is arranged rotatably around the hinge axis (6) of the hinge pin component (10) and is fixable in any desired position by means of a fixation device (8).

2. Bone gripping pincers according to claim 1, characterized in that at least one of the jaws (2,3) is a spring (15,16).

3. Bone gripping pincers according to claim 1 or 2, characterized in that the two legs (4,5), preferably in the form of handles, are removably mounted on the hinge (1).

4. Bone gripping pincers according to one of the claims 1 to 3, characterized in that the free ends of the two jaws (2,3) have sharp tips.

5. Bone gripping pincers according to one of the claims 1 to 4, characterized in that the locking device (7) is a locking nut.

6. Bone gripping pincers according to claim 5, characterized in that the hinge pin component (10) and the hinge bore component (20) are each provided with two concentrically offset movement stops (11,12;13,14), which limit reciprocal rotation of the two components (10,20) in the positive and negative direction.

7. Bone gripping pincers according to one of the claims 1 to 6, characterized in that the fixation device (8) is a fixation nut.

8. Osteosynthetic attachment device comprising at least two bone gripping pincers according to one of the claims 1 to 7, characterized in that the single bone gripping pincers are connected with each other by means of its connection pins (9).

## Revendications

1. Pince de saisie de l'os, comportant
A) une articulation (1) comportant un élément axial (10) de l'articulation et un élément alésé (20) de l'articulation correspondant à l'élément axial (10) de l'articulation, l'élément axial (10) de l'articulation portant un axe de l'articulation (6) cylindrique et disposé axialement;
B) deux lèvres (2, 3);
C) deux bras (4, 5), et
D) un dispositif d'immobilisation (7) de l'articulation (1), par lequel la position de l'élément axial (10) de l'articulation peut être immobilisée de manière libérable par rapport à l'élément alésé (20) de l'articulation;
caractérisée en ce que
E) le dispositif d'immobilisation (7) s'engage directement sur l'axe (6) cylindrique de l'articulation de l'élément axial (10) de l'articulation;
F) il est prévu une tige de liaison (9) par laquelle on peut réaliser une liaison avec d'autres pinces de saisie de l'os, et en ce que
G) la tige de liaison (9) est disposée à rotation autour de l'axe (6) de l'élément axial (10) de l'articulation et peut être bloquée dans toute position quelconque au moyen d'un dispositif d'immobilisation (8).

2. Pince de saisie de l'os selon la revendication 1, caractérisée en ce qu'au moins une des lèvres (2; 3) présente une configuration élastique (15; 16).

3. Pince de saisie de l'os selon la revendication 1 ou 2, caractérisée en ce que les deux bras (4, 5), de préférence configurés comme poignées, peuvent être enlevés de l'articulation (1).

4. Pince de saisie de l'os selon l'une des revendications 1 à 3, caractérisée en ce que l'extrémité libre des deux lèvres (2, 3) est configurée en pointe.

5. Pince de saisie de l'os selon l'une quelconque des revendications 1 à 4, caractérisée en ce que le dispositif d'immobilisation (7) est un écrou d'immobilisation.

6. Pince de saisie de l'os selon la revendication 5, caractérisée en ce que l'élément axial (10) de l'articulation et l'élément alésé (20) de l'articulation sont dotés chacun de deux limiteurs de déplacement (11, 12; 13, 14) concentriques, disposés décalés l'un par rapport à l'autre, qui limitent la rotation mutuelle des deux éléments (10, 20) dans le sens positif et dans le sens négatif de la rotation.

7. Pince de saisie de l'os selon l'une des revendications 1 à 6, caractérisée en ce que le dispositif d'immobilisation (8) est un écrou d'immobilisation (8).

8. Dispositif d'immobilisation pour ostéosynthèse comportant au moins deux pinces de saisie de l'os selon l'une des revendications 1 à 7, caractérisé en ce que les pinces individuelles de saisie de l'os sont reliées l'une à l'autre par leur tige de liaison (9).
